# EUROPEAN PATENT APPLICATION

(11) **EP 2 932 996 A1**
(43) Date of publication of application: **21.10.2015**
(21) Application number: 13890218.4
(22) Date of filing: 12.11.2013
(51) Int. Cl.: A61M 25/10

(54) **CONTACT-LIMITING SIDE BALLOON DOUBLE-HOLE URINARY CATHETER**

(30) Priority: 24.07.2013 CN 201310322421
(71) Applicant: Wang, Liang, Xiacheng Di, Zhejiang 310014 (CN)
(72) Inventor: Wang, Liang, Xiacheng Di, Zhejiang 310014 (CN)
(74) Representative: TLIP Limited
(86) International application number: PCT/CN2013/086922
(87) International publication number: WO 2015/010391

(57) **Abstract**

The invention relates to a contact-limiting side balloon double-hole urinary catheter, pertaining to the field of medical auxiliary devices; wherein a catheter body is made from a polymer blend, a side balloon is provided at one side of the catheter body close to a tip, the other side is provided with an excretion cavity drainage lower hole and an upper hole, and a flushing lumen liquid flow outlet is provided at the tip. After the urinary catheter is placed in the bladder, the side balloon automatically tilts in a water or gas injection state, the balloon wall and the catheter body do not press the trigone of the bladder and the orificium ureteris, the excretion cavity drainage lower hole is adjacent to the orificium ureteris to completely empty the bladder, and there is no residual urine. The tip, the side balloon, the excretion cavity drainage lower hole and upper hole, the excretion cavity, a water or gas injection lumen, the flushing lumen, the flushing lumen liquid flow outlet, an anti-reflux valve, an excretion cavity conical interface, a flushing cavity conical interface and a flushing cavity clipping switch of the urinary catheter are connected into an integral piece via the catheter body, and have a significant effect in clinical application.

## Description

### TECHNICAL FIELD

The invention relates to a contact-limiting side balloon double-hole urinary catheter, pertaining to the field of medical auxiliary devices.

### BACKGROUND OF THE INVENTION

In the medical field, a urinary catheter needs to be inserted for retention catheterization for curing internal medicine diseases, surgical diseases, obstetrical and gynecological diseases, orthopedic diseases and urologic diseases. The urinary catheter is a frequently-used medical auxiliary device, which may be inserted into a bladder via a patient's urethra to guide urine to flow out. After the urinary catheter is inserted into the bladder, a balloon is provided at a head end close to the urinary catheter to ensure fixed retention of the urinary catheter in the bladder, a drainage part of the urinary catheter is connected to a urine bag for collecting urine. The indwelling catheter has common problems such as urinary tract infection, inadequate drainage, difficult decannulation, bladder spasm or contracture and the like, but the most common complication is urinary infection. A triangular region of an interior surface of a fundus vesicae urinariae is disposed between two orificium ureters and an internal urethral orifice. The trigone of the bladder is a predilection site of tumour, tuberculosis and inflammation, being a key area in cystoscopy, and having important clinical significance.

A distance of 3-5cm is provided between an excretion cavity drainage hole of the existing balloon three-cavity urinary catheter and the fundus of bladder, and a balloon is provided in interval, partial residual urine is always left on the fundus of bladder; urine in the bladder generally is bacteria-free. However, urine is an environment suitable for breeding bacteria. If urine is unable to be completely emptied and left on the fundus of bladder, inflammation may easily occur due to arbitrary generation of bacteria. The urinary catheter (as a foreign body) stays in the bladder on a long-term basis and contacts residual urine, which may easily deposit urate calculi in the tail end of the ureter, thus forming calculus. The balloon is shaped like a round when it is filled with water or air, the balloon wall continuously irritates the trigone of the bladder, which may induce inflammation, the patient may feel frequency/urgency of urination and feel subabdominal discomfort; leakage of urine may be resulted from urethral relaxation, and leakage of urine may be resulted from relaxation of pelvic floor muscle and urethral sphincter of elderly female patients suffering from intracranial lesions or paraplegia; in the method for using a traditional urinary catheter, water or gas injection is unavailable until the urinary catheter is further inserted by about 3cm after urine is seen.

### SUMMARY OF THE INVENTION

The object of the invention is to overcome defects that after the existing balloon three-cavity urinary catheter is inserted into human body, water or gas injection is unavailable until the urinary catheter is further inserted by about 3cm after urine is seen, a distance of 3-5cm is provided between an excretion cavity drainage hole of the balloon urinary catheter and the fundus of bladder and a balloon is provided in interval, partial residual urine is always left on the fundus of the bladder, which may breed bacteria, lead to inflammation, form calculus and result in leakage of urine; the trigone of the bladder is continuously irritated by the balloon wall, which may induce inflammation, the patient may feel frequency/urgency of urination and feel subabdominal discomfort; a contact-limiting side balloon double-hole urinary catheter is provided, it is available for water or gas injection after the urinary catheter is inserted into the bladder, fixed firmly, no irritation on the trigone of the bladder, no residual urine, no irritation sign of bladder, and no leakage of urine, thus removing various complications produced by the existing balloon urinary catheter.

In order to achieve the foregoing object, the invention adopts such a technical solution as below: a contact-limiting side balloon double-hole urinary catheter, including a catheter body, a tip, a side balloon, a balloon cavity, an excretion cavity drainage upper hole, an excretion cavity, a water or gas injection lumen communicated with the balloon, an excretion cavity drainage lower hole, a balloon through hole of the water or gas injection lumen communicated with the balloon, a flushing lumen, a flushing lumen liquid flow outlet, an anti-reflux valve for water or gas injection of the balloon, an excretion cavity conical interface, a flushing cavity conical interface and a flushing cavity clipping switch; wherein the catheter body is made from a polymer blend, the side balloon of the urinary catheter is provided at one side of the catheter body close to the tip, the excretion cavity drainage lower hole is provided on the bottom of the balloon close to an opposite side of the catheter body of the side balloon of the urinary catheter, the excretion cavity drainage upper hole is provided on a top close to the balloon, the flushing lumen liquid flow outlet is disposed on the tip, and is arranged on an inclined plane of the catheter body at either side of a plane formed by the excretion cavity drainage upper hole, the excretion cavity drainage lower hole and the balloon through hole of the water or gas injection lumen communicated with the balloon; a use method of the urinary catheter is as below: water or gas injection is available as along as urine is provided, after the urinary catheter is placed in the bladder, the side balloon automatically tilts in a water or gas injection state, a space frame comprising the side balloon tilted, the urinary catheter and the bladder wall bottom is large enough to accommodate the trigone of the bladder, neither the bladder wall nor the catheter body contacts the trigone of the bladder or a little contact is possible so as to continuously fix the urinary catheter in the bladder; after the urinary catheter is placed in the bladder, the side balloon does not press the trigone of the bladder and the orificium ureteris in a water or gas injection state; after the urinary catheter is placed in the bladder, the excretion cavity drainage lower hole is adjacent to the orificium ureteris to completely empty both normal urine and abnormal urine in the bladder, and there is no residual urine in the bladder. The tip, the side balloon, the balloon cavity, the excretion cavity drainage upper hole, the excretion cavity, the water or gas injection lumen communicated with the balloon, the excretion cavity drainage lower hole, the balloon through hole of the water or gas injection lumen communicated with the balloon, the flushing lumen, the flushing lumen liquid flow outlet, the anti-reflux valve for water or gas injection of the balloon, the excretion cavity conical interface, the flushing cavity conical interface and the flushing cavity clipping switch are connected into an integral piece via the catheter body.

Further, after the urinary catheter is placed in the bladder and the side balloon is in a water or gas injection state, as the excretion cavity drainage lower hole is adjacent to the orificium ureteris, both normal urine and abnormal urine in the bladder may be completely emptied by means of absorption, negative pressure and siphoning or the like, and there is no residual urine.

Further, the catheter body part of the urinary catheter is made from an extrudable and mouldable material by means of injection molding, extrusion moulding and/or blow molding, or a combination of adhesion and bonding, for example, a silicagel, or a silica-gel mixture, or an latex mixture, or a polyolefin such as polyethylene (PE) or polypropylene (PP), or a copolymer of PE such as ethylene vinyl acetate (EVA) or polyvinyl chloride (PVC) or PVDC. By using the materials required, the tip of the urinary catheter, the side balloon, the balloon cavity, the excretion cavity drainage upper hole, the excretion cavity, the water or gas injection lumen communicated with the balloon, the excretion cavity drainage lower hole, the balloon through hole of the water or gas injection lumen communicated with the balloon, the flushing lumen, the flushing lumen liquid flow outlet, the anti-reflux valve for water or gas injection of the balloon, the excretion cavity conical interface, the flushing cavity conical interface and the flushing cavity clipping switch are made into an integral piece.

Further, in order to obtain good mechanical properties and enough rigidity for intended use, a thorough sterilization may be achieved by known sterilization methods, materials for manufacturing and constituting the catheter body part of the urinary catheter have a shore hardness A ranged from 75 to 85, preferably ranged from 78 to 82, have radiation resistance so that they are capable of resist to at least 50kGy without degradation basically, and have a melting temperature of more than 90°C, preferably more than 110°C and the most preferably more than 130°C; and materials for manufacturing and constituting the catheter body part of the urinary catheter have low elasticity, good twist performance and good dimensional stability.

Further, the polymer blend includes polyolefin having a weight percentage of at least 80%, which is blended with medical oil and/or paraffin.

Further, a tail pipe of the anti-reflux valve for water or gas injection of the balloon and the flushing cavity clipping switch are made from rigid material, and the flushing cavity clipping switch may be separated from the catheter body.

The invention has such beneficial effects as below: the tip of the urinary catheter is smooth, water or gas injection is available as along as urine is provided after the contact-limiting side balloon double-hole urinary catheter is placed in a patient's bladder; the side balloon of the head end swells and automatically tilts after water or gas injection, a space frame comprising the side balloon tilted, the urinary catheter and the bladder wall bottom is large enough to accommodate the trigone of the bladder, neither the bladder wall nor the catheter body contacts the trigone of the bladder or a little contact is possible so as to continuously fix the urinary catheter in the bladder; the side balloon does not press the trigone of the bladder and the orificium ureteris at both sides, the excretion cavity drainage lower hole is adjacent to both the orificium ureters; both normal urine and abnormal urine in the bladder may be completely emptied by means of absorption, negative pressure and siphoning or the like, and there is no residual urine in the bladder. The flushing lumen liquid flow outlet is disposed on an inclined surface of the catheter body at either side of the tip, and is communicated with the flushing cavity conical interface through the flushing lumen, the flushing cavity conical interface may act as a connector of the urinary catheter and may be connected to other devices; the flushing cavity clipping switch is disposed at the flushing cavity conical interface and may completely cut off the flushing lumen, rinsing solution may be injected into the bladder through the channel, and urine may be guided and drained in emergency if necessary. Patients feel no pain and remarkable effects may be achieved in clinical application.

### BRIEF DESCRIPTION OF THE DRAWINGS

Further description of the invention is made by reference to brief description of the drawings and detailed description of the embodiments.
Fig. 1 is a structural schematic diagram of the contact-limiting side balloon double-hole urinary catheter of the invention after water or gas injection of the balloon.
Fig. 2 is a structural schematic diagram of a longitudinal section of the contact-limiting side balloon double-hole urinary catheter of the invention where the head end of the catheter body passes through a center of the water or gas injection lumen communicated with the balloon.
Fig. 3 is a structural schematic diagram of a longitudinal section of the contact-limiting side balloon double-hole urinary catheter of the invention where the head end of the catheter body passes through a center of the flushing lumen.
Fig. 4 is a structural schematic diagram (after water or gas injection, enlarged) of a cross section of the contact-limiting side balloon double-hole urinary catheter of the invention where the catheter body passes through a center of the excretion cavity drainage upper hole.
Fig. 5 is a structural schematic diagram (after water or gas injection, enlarged) of a cross section of the contact-limiting side balloon double-hole urinary catheter of the invention where the catheter body passes through a center of the balloon through hole of the water or gas injection lumen communicated with the balloon.
Fig. 6 is a structural schematic diagram (after water or gas injection, enlarged) of a cross section of the contact-limiting side balloon double-hole urinary catheter of the invention where the catheter body passes through a center of the excretion cavity drainage lower hole.
Fig. 7 is a structural schematic diagram (no water or gas injection, enlarged) of a cross section of the contact-limiting side balloon double-hole urinary catheter of the invention where the catheter body passes through a center of the balloon through hole of the water or gas injection lumen communicated with the balloon.
Fig. 8 is a structural schematic diagram (enlarged) of a cross section of the catheter body D of the contact-limiting side balloon double-hole urinary catheter of the invention according to Fig. 9.
Fig. 9 is a structural schematic diagram of the tail end of the contact-limiting side balloon double-hole urinary catheter of the invention.

In Figs., 1-catheter body, 2-tip, 3-balloon cavity, 4-excretion cavity drainage upper hole, 5-excretion cavity, 6-water or gas injection lumen communicated with the balloon, 7-excretion cavity drainage lower hole, 8-balloon through hole of the water or gas injection lumen communicated with the balloon, 9-flushing lumen, 10-flushing lumen liquid flow outlet, 11-anti-reflux valve for water or gas injection of the balloon, 12-excretion cavity conical interface, 13-flushing cavity conical interface, 14-flushing cavity clipping switch, 15-side balloon, A-cross section of the catheter body passing through a center of the excretion cavity drainage upper hole; A-cross section of the catheter body passing through a center of the excretion cavity drainage upper hole; B-cross section of the catheter body passing through a center of the balloon through hole of the water or gas injection lumen communicated with the balloon; C-cross section of the catheter body passing through a center of the excretion cavity drainage lower hole; D-cross section of the catheter body; E-longitudinal section of the head end of the catheter body passing through a center of the flushing lumen; F-longitudinal section of the head end of the catheter body passing through a center of the water or gas injection lumen communicated with the balloon.

### DETAILED DESCRIPTION OF THE EMBODIMENTS

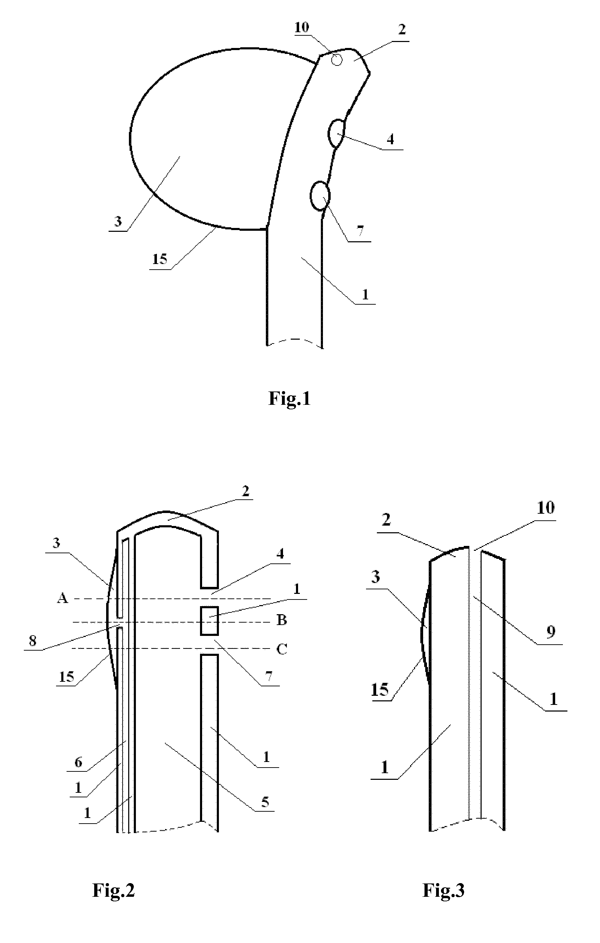

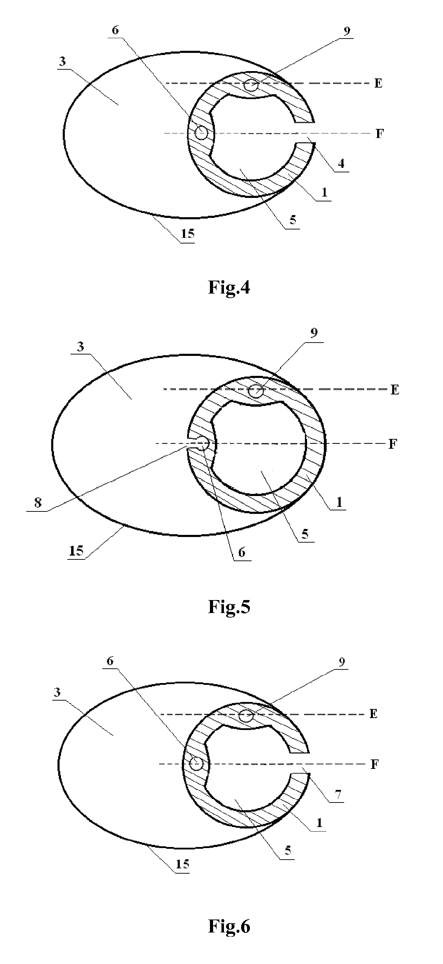

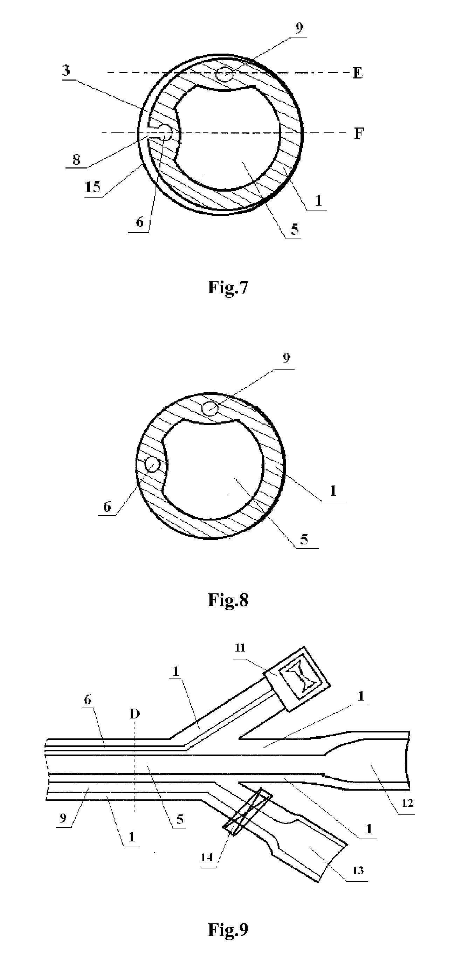

In Figs. 1-9, a contact-limiting side balloon double-hole urinary catheter, including a catheter body 1, a tip 2, a side balloon 15, a balloon cavity 3, an excretion cavity drainage upper hole 4, an excretion cavity 5, a water or gas injection lumen communicated with the balloon 6, an excretion cavity drainage lower hole 7, a balloon through hole of the water or gas injection lumen communicated with the balloon 8, a flushing lumen 9, a flushing lumen liquid flow outlet 10, an anti-reflux valve for water or gas injection of the balloon 11, an excretion cavity conical interface 12, a flushing cavity conical interface 13 and a flushing cavity clipping switch 14; wherein the side balloon 15 of the urinary catheter is provided at one side of the catheter body 1 close to the tip 2, the excretion cavity drainage lower hole 7 is provided on the bottom of the balloon 15 close to an opposite side of the catheter body 1 of the side balloon 15 of the urinary catheter, the excretion cavity drainage upper hole 4 is provided on a top close to the balloon 15, the flushing lumen liquid flow outlet 10 is disposed on the tip 2, and is arranged on an inclined plane of the catheter body 1 at either side of a plane formed by the excretion cavity drainage upper hole 4, the excretion cavity drainage lower hole 7 and the balloon through hole of the water or gas injection lumen communicated with the balloon 8.

After the contact-limiting side balloon double-hole urinary catheter is placed in a patient's bladder, water or gas may be injected by an injection syringe to the side balloon cavity 3 through the anti-reflux valve for water or gas injection of the balloon 11 as along as urine is provided; bacteria-free water or gas enters into the water or gas injection lumen communicated with the balloon 6, the side balloon 15 of the head end automatically tilts after water or gas injection, a swelling balloon is formed in the bladder, an obstacle is formed, the urinary catheter is fixed so as to prevent the contact-limiting side balloon double-hole urinary catheter from getting out of a urethra, a space frame comprising the tilted side balloon 15 supported by the catheter body 1 of the urinary catheter and the bladder wall bottom is large enough to accommodate the trigone of the bladder and the excretion cavity drainage lower hole 7, the bladder wall does not contact the trigone of the bladder or a little contact is possible so as to continuously fix the urinary catheter in the bladder; the side balloon does not press the trigone of the bladder and the orificium ureteris at both sides, the excretion cavity drainage lower hole 7 is adjacent to both the orificium ureters; both normal urine and abnormal urine in the bladder may be completely emptied through the excretion cavity 5 and the excretion cavity conical interface 12 by means of absorption, negative pressure and siphoning or the like, and there is no residual urine in the bladder. The excretion cavity conical interface 12 may act as a connector of the urinary catheter and may be connected to other devices such as a collection bag, a drainage tube or an injection syringe, etc. The flushing lumen liquid flow outlet 10 is disposed on an inclined surface of the catheter body 1 at either side of the tip 2, and is communicated with the flushing cavity conical interface 13 through the flushing lumen 9, the flushing cavity conical interface 13 may act as a connector of the urinary catheter and may be connected to other devices; the flushing cavity clipping switch 14 is disposed at the flushing cavity conical interface 13 and may completely cut off the flushing lumen when in use, rinsing solution may be injected into the bladder through the channel, and urine may be guided and drained in emergency if necessary. Patients feel no pain and remarkable effects may be achieved in clinical application.

It is to be explained at last that the above embodiments are only for description of the technical scheme of the invention instead of restriction. Although a detailed description of the invention is made by referring to preferred embodiments,those of ordinary skill in the art shall understand that it is possible to modify or equivalently replace the technical scheme of the invention without departing from the aim and scope of the technical scheme of the invention, which shall be within the scope of claims of the invention.

## Claims

1. A contact-limiting side balloon double-hole urinary catheter, comprising a catheter body, a tip, a side balloon, a balloon cavity, an excretion cavity drainage upper hole, an excretion cavity, a water or gas injection lumen communicated with the balloon, an excretion cavity drainage lower hole, a balloon through hole of the water or gas injection lumen communicated with the balloon, a flushing lumen, a flushing lumen liquid flow outlet, an anti-reflux valve for water or gas injection of the balloon, an excretion cavity conical interface, a flushing cavity conical interface and a flushing cavity clipping switch; wherein the catheter body is made from a polymer blend, the side balloon of the urinary catheter is provided at one side of the catheter body close to the tip, the excretion cavity drainage lower hole is provided on the bottom of the balloon close to an opposite side of the catheter body of the side balloon of the urinary catheter, the excretion cavity drainage upper hole is provided on a top close to the balloon, the flushing lumen liquid flow outlet is disposed on the tip of the urinary catheter, and is arranged on an inclined plane of the catheter body at either side of a plane formed by the excretion cavity drainage upper hole, the excretion cavity drainage lower hole and the balloon through hole of the water or gas injection lumen communicated with the balloon; the tip of the urinary catheter, the side balloon, the balloon cavity, the excretion cavity drainage upper hole, the excretion cavity, the water or gas injection lumen communicated with the balloon, the excretion cavity drainage lower hole, the balloon through hole of the water or gas injection lumen communicated with the balloon, the flushing lumen, the flushing lumen liquid flow outlet, the anti-reflux valve for water or gas injection of the balloon, the excretion cavity conical interface, the flushing cavity conical interface and the flushing cavity clipping switch are connected into an integral piece via the catheter body.

2. The contact-limiting side balloon double-hole urinary catheter according to claim 1, wherein after the urinary catheter is placed in the bladder and the side balloon is in a water or gas injection state, the excretion cavity drainage lower hole is adjacent to the orificium ureteris to empty the bladder, and there is no residual urine.

3. The contact-limiting side balloon double-hole urinary catheter according to claim 1 or 2, wherein the catheter body is made from an extrudable and mouldable material.

4. The contact-limiting side balloon double-hole urinary catheter according to claim 1 or 2, wherein a tail pipe of the anti-reflux valve for water or gas injection of the balloon and the flushing cavity clipping switch are made from rigid material, and the flushing cavity clipping switch may be separated from the catheter body.

5. The contact-limiting side balloon double-hole urinary catheter according to claim 1 or 2, wherein the polymer blend comprises polyolefin having a weight percentage of at least 80%, which is blended with medical oil and/or paraffin.

6. The contact-limiting side balloon double-hole urinary catheter according to claim 1 or 2, wherein the catheter body has a shore hardness A ranged from 75 to 85.

7. The contact-limiting side balloon double-hole urinary catheter according to claim 1 or 2, wherein the catheter body has radiation resistance so that it may resist to at least 50kGy without degradation basically.

8. The contact-limiting side balloon double-hole urinary catheter according to claim 1 or 2, wherein the catheter body has a melting temperature of above 90°C.
